# EUROPEAN PATENT SPECIFICATION

(11) **EP 3 320 889 B1**
(45) Date of publication and mention of the grant of the patent: **16.12.2020**
(21) Application number: 16832377.2
(22) Date of filing: 06.07.2016
(51) Int. Cl.: A61H 15/00, A61H 11/00, A61H 7/00, A61H 39/04, A61H 23/02, A61N 2/00, A61N 5/06, A61H 1/02, A61H 23/00, A61F 7/00, A61N 1/36, A61N 1/04, A61H 39/08

(54) **MASSAGE DEVICE FOR ACTING ON THE SPINAL MUSCLES OF THREE REGIONS OF THE SPINE**
MASSAGEVORRICHTUNG ZUR WIRKUNG AUF DIE WIRBELSÄULENMUSKELN VON DREI BEREICHEN DER WIRBELSÄULE
APPAREIL DE MASSAGE POUR AGIR SUR LES MUSCLES DU DOS DE TROIS RÉGIONS DE LA COLONNE VERTÉBRALE

(30) Priority: 10.07.2015 RU 2015127903
(43) Date of publication of application: 16.05.2018
(73) Proprietor: Fortis Axis, Uzdaroji Akcine Bendrove, 09308 Vilniaus (LT)
(72) Inventor: SITSIHOVSKIY, Pavel Victorovich, Moscow 107207 (RU); BAGRINOVSKIY, Aleksey Borisovich, Ekaterinburg 620146 (RU); GONCHAROV, Igor Georgievich, Ekaterinburg 620130 (RU)
(74) Representative: Spengler, Robert
(86) International application number: PCT/IB2016/001648
(87) International publication number: WO 2017/021784

(56) References cited:
- WO-A2-2009/022341
- RU-C1- 2 141 300
- RU-U1- 148 304
- US-A- 3 705 579
- US-A- 4 945 900

## Description

### TECHNOLOGICAL AREA OF APPLICATION

This invention relates to physiotherapy and can be used as a self-massage device in exercises included in health and fitness exercise programs.

### TECHNOLOGICAL LEVEL

Document RU 148 304 U1 discloses a device for massaging muscles and BAP of the paravertebral region of the spine, containing a base with massage elements located on it and additional massage elements located on the side surface of the base. The base is made of a solid inelastic material of ellipsoid shape with a supporting lower part in the form of three flat surfaces, with the middle supporting part protruding by 5-7 mm and made of elastic material, and the massaging elements are made in the form of a pyramid with rounded peaks and ribs, the distance between which is 75-85 mm, and additional paired massaging elements are made in the form of pyramids with rounded peaks and faces with a distance between the peaks of 45-55 mm.

Document US 4,945,900 discloses a roller massaging apparatus performing massage by applying pushing pressure on a human body by means of a pushing pressure roller.

Document RU 2 141 300 C1 discloses a device for self-massage of deep muscles of back and neck. The device has a case with massaging members arranged on it. Spaces between them correspond to boundaries of the paravertebral region of thoracic and lumbar sections of the vertebral column. The massaging members are coupled to one another over a curvilinear surface.

Deep-lying spinal muscles (paravertebral muscles) are slow-type fibre and static muscles. Due to their structure, they are not capable of immediate relaxation without assistance. A spasm of deep muscles simultaneously triggers off a spasm of blood vessels, hindering blood circulation and causing osteochondrosis.

It is known that different kinds of mechanical manipulation of corset muscles have a positive effect on the spine and thus on general health of a person.

Massage devices are used for relaxation of deep muscles because they exert a multifunctional effect on the spinal regions and relieve spinal muscles off hypertonus and restore their normal functions.

There is a device for correcting spine and paravertebral muscles (RF Patent No. 2465881). That device carries three pairs of massaging heads intended for relaxing the cervical, thoracic and lumbar regions of the spine.

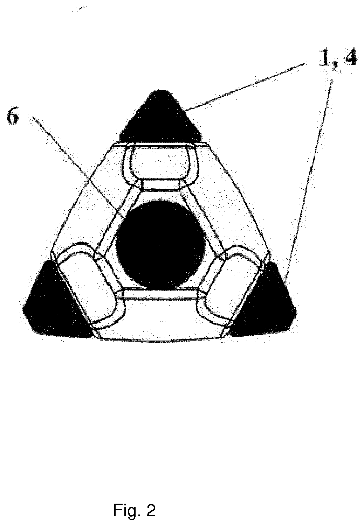

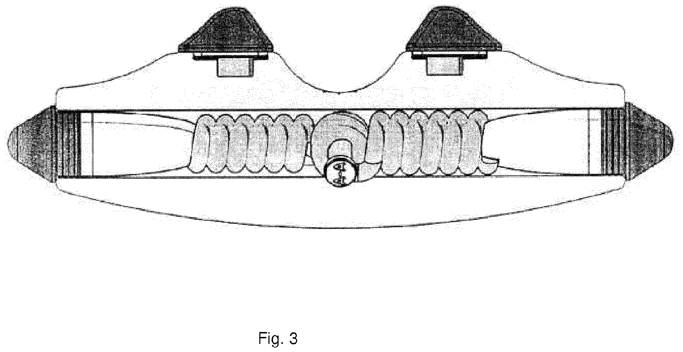

The massager used for correcting the spine and relieving paravertebral muscles (RF Patent for a utility model No. 144668) is closest to the present invention. That device comprises three pairs of massaging heads, each intended for a different spinal region. The bases of each pair of heads represent crescent-shaped recesses. They are spaced at a sufficient distance from each other, at a depth from their vortices so that spinous processes of vertebrae can be accommodated in these recesses (see Figs. 2 and 3 of the patent mentioned). The vortices of the massaging pairs are removed from each other at a distance commensurate with the features of the vertebrae of each particular region, while the distance between the massaging pairs is appropriate for producing a correcting effect for each spinal region, taking into account the anatomy of each region.

Both existing models belong to three-position massagers for paravertebral spinal muscles in the sense that, as their bodies are based on a three-sided prism with ribs distorted in such a fashion as to approximate them to the shape of the surface of the back between two adjacent spinous processes of the spine when the rib is positioned vertically with respect to the spinal column; when the massager is turned over, the ribs of the body of the massager located opposite the side on which that body is lying, come to the top. As the ribs are made of different shape, in correspondence with the different spine segments, the impact is adapted to the shape of the back in different regions of the spine.

The existing solutions display the following weaknesses, which prevent achievement of the desired technological result.
1. Paravertebral muscles undergo effect of working heads formed by the body of the device and cannot be detached from its body. Consequently, hardness of the massage components is determined by the hardness of the body material and cannot be changed in accordance with the impact required.
2. The impact of working components on massaged areas is determined by the person's weight. That impact can only be controlled by the person's movements, which prevents complete relaxation of the muscle carcass.
3. Geometry of the said massagers is limited by the three sizes of the working vortices and the distances between the working vortices, conventionally calculated for the effect on the three spinal regions: cervical, thoracic and lumbar. Conventional subdivision does not allow for adjusting distances between the vortices individually for each person, or accounting for differences between male and female anatomies or between different persons of the same gender.
4. Moving the massagers discussed to another region of the spine of a lying down person is difficult because these massagers are not equipped with handles that would help to move them along the spine column.

The above-mentioned drawbacks prevent the existing massagers from being comfortably, effectively and safely, with no medical supervision, used as sports implement for exercises aimed at relaxation of deep paravertebral muscles in persons of different build.

Spasm-affected or stressed muscles require soft, gentle, controllable massaging components. The above-discussed massagers cause some amount of pain as well as certain other physiological discomfort, which might preclude their application for a period of time required for a particular treatment.

The lack of control does not permit gradual increase of effort as might be desirable for a particular exercise program.

The lack of adjustment of the massager for a particular person means that working vertices might not be applied to the required area.

### INVENTION DESCRIPTION

This invention aims at producing a massager for acting physically on the muscles and legaments of each region of the spine individually, while allowing the user to move the massager along the spine column without assistance, use the massager in an standing upright position and adjust easily intensity, direction and depth of massage according to one's physiological characteristics.

Technological results ensured by this invention consist in that, thanks to detacheable massage heads and using a range of detacheable massage heads of different shape, size and hardness, a greater precision of controlling stress application, depth and direction of massage therapy applied to paravertebral muscles has been achieved, whatever physiological characteristics the user has (i.e. the proposed massager is universal; no need to make different massagers for persons with different physiological characteristics).

This goal was achieved by making a massager intended for muscles of at least three spine regions include the following components:
detachable massage heads;
a body with sockets to hold the above massage heads;
the said body has three sides, each of the sides ensuring a firm installation of the body on a horizontal surface, and the planes tangential to each of the said sides and coinciding with the said horizontal plane where a firm installation of each side is ensured, form with one another a two-sided angle of approximately 60± 15 degrees, wile the sum total of all the two-sided angles equals 180 degrees;
the said sides of the body link with one another by the means of three rib surfaces, each of the said three rib surface, each of which carries lateral parts for paravertebral regions of the spine, and a medial part for the spinal column, made sunk along the longitudinal axis of the body with relative to the said lateral parts;
the said sockets are located in the lateral parts of each of the said rib zones;
the said three rib surfaces, together with the said massage heads secured in the sockets, form massage surfaces corresponding to the position of the massaged organs of the three different spinal regions of the back;
the but ends of the body carry sockets where handles and/or plugs can be secured.

Preferentially, to have the said sockets capable of holding massage heads with different coefficient of elasticity.

It is especially preferable to have the said sockets suited for holding massage heads of different geometry on each side.

In one of the versions of the massager, the said plugs are made of an elastic material. Plugs can be designed as an extra massage piece. Plugs of this kind can be used as massage pieces for local massage of various muscle segments.

It is advisable to have the said handles of at least 20 cm long so that they could be gripped by hands. With handles of this kind, one can use the massager when standing upright or lying down in a horizontal position.

It may be desirable in some cases to make the said body hollow and to make the said sockets on the butt sides, going right through the wall, linked to the cavity in the body and to run a rope through the body, and the ends of said rope be at least 20 cm long from the sockets to the lugs made to be gripped by hands. This arrangement allows the user to move the massager with ease when in a lying down position.

In one of the versions, when the said body is made hollow, a permanent magnet can be placed inside the cavity- to have a magnetic field acting on the person's body.

In another version, when the said body is hollow, a vibrating device can be installed inside the cavity to cause vibration to the body.

In yet another version, when the said body is made hollow, a generator of reciprocal vibrations of the said massaging heads can be installed inside the cavity.

In one of the most preferential versions the massager is equipped with a set of massage heads of different shape and size and/or with different coefficient of elasticity. The user can chose any head from the set and secure it in an appropriate socket, thus adjusting intensity of impact on one or another spine region, or else adjusting the size of the heads in accordance with their own physiological characteristics.

The massager aims at eliminating spasms of deep paravertebral muscles caused by strenuous sports activities.

The massager is best suited for use in exercises for general wellbeing and fitness purposes, in therapeutic exercises, sports, for reducing hypertension in paravertebral muscular tissue.

### BRIEF DESCRIPTION OF DRAWINGS

Fig. 1 shows a diagram of the massager in accordance with one of its preferential versions.
Fig. 2 shows a diagram of the same massager as shown in Fig. 1 as viewed from one of the butt ends.
Fig. 3 shows a diagram of the same massager, sectioned.
Fig. 4 shows the same massager foreshortened ¾.
Fig. 5 shows an assembly drawing of the same massager.
Fig. 6 shows a diagram of the massager manufactured in accordance with one of the preferred versions, with handles secured in butt sockets. For simplicity purpose, detachable components are not shown.

### REALISATION OF INVENTION

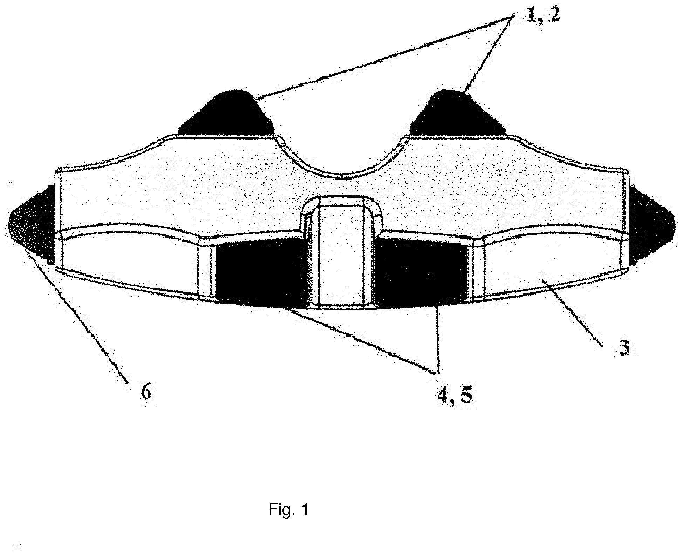

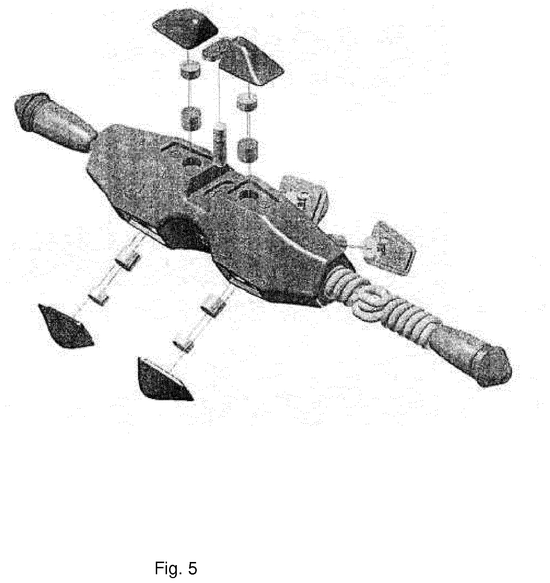

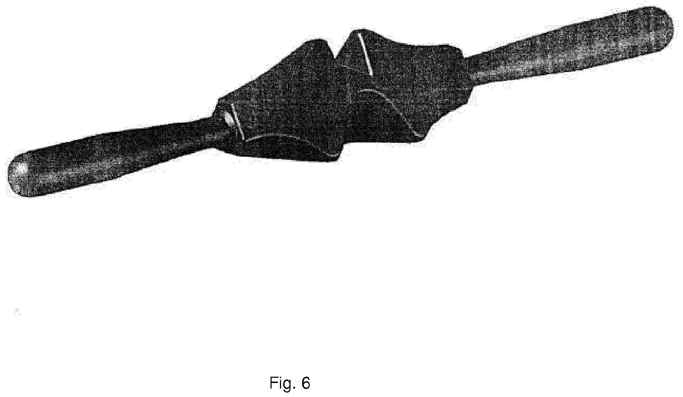

As shown in Figs. 1-6, the massager for paravertebral muscles of the back, for at least three regions of the spine, comprises the following parts:
detachable massage heads 1, 2, 4, and 5;
body 3 with sockets for the said massage heads;
the said body 3 has three sides, each of which ensures firm installation of the body on a horizontal surface, and the planes tangential to each of the said sides and coinciding with the said horizontal surface, on which firm installation of each side is secured, form with one another a two-sided angle of approximately 60±15 degrees, while the sum total of all the two-sided angles amounts to 180 degrees;
the said sides of the body 3 are conjugated with one another by the means of three rib surfaces, each of which has lateral parts for paravertebral regions of the back and a medial part for the spine column, sunk along the longitudinal axis of the body with relative to the said lateral parts;
the said sockets are arranged in the lateral parts of each of the said rib zones;
the said three rib surfaces, together with the said massage heads form massage surfaces corresponding to position of massaged organs of the three regions of the spine;
the butt ends of the body carry sockets that give an opportunity of securing handles and/or plugs 6.

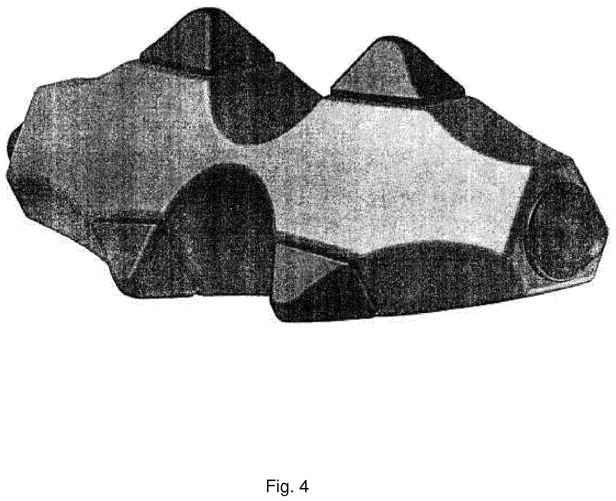

As shown in Figs. 2 and 4, the body of the massager is similar in shape to a six-sided prism. The three non-adjacent sides of the prism carry three slots where three pairs of working heads can be secured. The prism is manufactured, using the method of intersection of three spheres, resulting in the formation of a three-sided figure, which has three ribs and three sides. The six-sided prism formed by truncation of the ribs of the three-sided prism. The sides of the body have a complex spherical shape, which allows the massager standing on a surface to have a degree of freedom in two planes with respect to the surface and to the axis vertical to the surface.

The body contains a longitudinal cavity open on both ends.

Massage heads are made out of elastic materials of different hardness. Pairs of heads are secured on an appropriate side. Massage heads can also be secured on a side that does not correspond to the profile of the head, or else heads can be secured randomly in order to select required distances between massage heads.

The butt plugs are connected with one another with a rope run through the body of the massager. The plugs taken out of the body together with the rope form a flexible component with lugs, can be used as a handle for moving the massager along the spine column in a lying down position.

When the plugs are secured, the rope is placed in the cavity in the body of the massager.

Butt end plugs carry a massage protuberance, designed for localised effect on muscular tissue.

To assemble the massager appropriate pairs of massage heads are secured in the slots of the body.

The assembled massager (with massage heads secured in place) has three spherical bases and three pairs of heads.

To use the massager, it is placed on an even horizontal surface with one of the spherical sides down in such a fashion that one of the sides of the body faces upwards.

The user, in a lying down position, leans an elbow on the massager in such a fashion that one of the sides would rest against paravertebral muscles of the spine. The effect of massage on massaged areas is defined by the user's own weight, and it can be modified by installing heads of different hardness, thus increasing or decreasing the contact area.

There is an option of installing permanent magnets directly underneath the massage heads. Magnets generate a powerful magnetic field in the massage head/muscle tissue contact area, which generates a good therapeutic effect (magnetic therapy), producing the following results for example:
- stimulates blood flow, improves oxygen carrying capacity of blood;
- improves macro- and microrheology of blood by changing (usually reducing) viscosity;
- changing velocity of calcium ions, which produces two results: calcium reaches the damaged bone tissue (fractured for example) faster, speeding up its recovery, on the one hand, while on the other hand, calcium is washed out faster from an arthritic joint.

Using the massager equipped with detachable massage components exerts a careful and gentle effect while correcting impaired functions of the spine, eliminates hypertension in muscles, ligaments and joint capsules, restores mobility in each vertebral-motor segment, frees pinched spinal cord nerves. Delicate effect of the massager is ensured by the use of working heads of different hardness. The massager also exerts its effect on Shako's points, located symmetrically on both sides of the spinal column, which has a positive effect on the kidneys and urogenital system.

The deep massaging effect on paravertebral regions stimulates kinesthetic sensors of deep groups of muscles of the back, fascia, joints and ligaments of the spine, stretches out shrunk muscles and opens facet joints and improves their flexibility. Muscle tone of paravertebral muscles becomes normalized.

The massager works both on the segment and joint levels, which reduces tension in extensor muscles of the back and opens the blocked spinal region.

A deep effect on paravertebral regions, which represent reflex zones, through which zones functioning of internal organs is controlled, improves hemodynamics and trophism of the paravertebral zone.

Relieving myofascial pain syndrome significantly produces a significantly emphasised parasympathetic effect on cardiac function and hemodynamics, which is accompanied by decreased frequency of maladaptive reactions to an orthotest.

After a rehabilitation course with the use of the massager on paravertebral muscles, decreased blood pressure, tension index and calculated parameters of the heat index are observed.

Relief of paravertebral muscles with a massager reduces vertebrogenic pain syndrome and helps to prevent motor vegetative disorders under the condition of continuous building up the muscle corset with specialized physical exercises.

## Claims

1. A massager for back muscles of at least the three spinal regions cervical, thoracic and lumbar, comprising:
a body (3) having butt ends and three sides, each of which sides ensures installation of the massager on a horizontal surface, while the planes tangential to each of the said sides and coinciding with the said horizontal surface, which ensures installation of each of the sides, form with one another a two-sided angle of 60±15 degrees, while the sum total of all the two-sided angles comprises 180 degrees,
the said sides of the body (3) are conjugated with one another by the means of three rib surfaces, each of which carries lateral parts for paravertebral areas of the back and a medial part for the spinal column, made sunk along the longitudinal axis of the body (3) in relation to the lateral parts;
**characterized in that** the massager further comprises
detachable massage heads (1, 2, 4, 5), wherein
the body (3) is with sockets designed to secure the said massage heads (1, 2, 4, 5), and
the said sockets are located in the lateral parts of each of the said rib zones;
the said three rib zones, together with the said massage heads (1, 2, 4, 5) secured in the sockets, form massage surfaces corresponding to the positions of the massaged organs of the back in the three different regions of the spine; and wherein
the butt ends of the body (3) carry sockets where handles and/or plugs (6) can be secured.

2. A massager as in Claim 1, in which the said sockets can hold massage heads (1, 2, 4, 5) of different coefficient of elasticity.

3. A massager as in Claim 1, in which the said sockets can hold massage heads (1, 2, 4, 5) with different geometry each of each side.

4. A massager as in Claim 1 having plugs (6) secured at the sockets carried by the butt ends of the body (3), wherein the plugs (6) are made of elastic material.

5. A massager as in Claim 1 where the said handles are at least 20 cm long each and can be gripped by the hands.

6. A massager as in Claim 1 where the said body (3) is made hollow, while the said sockets on the butt ends go through the wall and link with the cavity in the body (3), so that a rope can be run through the body (3), the ends of which have lugs for the hands and are at least 20 cm long, measured from the sockets to the lug.

7. A massager as in Claim 1 where the said body (3) is made hollow and a permanent magnet is installed in the cavity.

8. A massager as in Claim 1 where the said body (3) is made hollow and a vibrating device is placed in the cavity, which can vibrate the body (3) of the massager.

9. A massager as in Claim 1 where the said body (3) is made hollow, and a device that causes reciprocal vibrations of the said massage heads (1, 2, 4, 5) is installed in the cavity.

10. A massager as in Claim 1, equipped with a set of massage heads (1, 2, 4, 5) of different shape, size and/or with different coefficients of elasticity.

## Patentansprüche

1. Massagegerät für die Rückenmuskulatur mindestens der drei Wirbelsäulenbereiche Hals-, Brust- und Lendenwirbelsäule, umfassend:
ein Hauptteil (3) mit verdickten Enden und drei Seiten, wobei jede der Seiten die Installation des Massagegeräts auf einer horizontalen Oberfläche gewährleistet, während die tangential zu jeder der Seiten verlaufenden, mit der horizontalen Oberfläche zusammenfallenden Ebenen, die die Installation jeder der Seiten gewährleisten, zusammen einen zweiseitigen Winkel von 60° ± 15° bilden, während die Summe aller zweiseitigen Winkel 180 ° umfasst,
die Seiten des Hauptteils (3) über drei Rippenflächen miteinander konjugiert sind, die jeweils seitliche Teile für paravertebrale Bereiche des Rückens und ein mediales Teil für die Wirbelsäule tragen, die entlang der Längsachse des Hauptteils (3) in Bezug auf die seitliche Teile versenkt ausgebildet sind;
**dadurch gekennzeichnet, dass** das Massagegerät ferner umfasst:
abnehmbare Massageköpfe (1, 2, 4, 5), wobei:
das Hauptteil (3) mit Buchsen versehen ist, die zur Befestigung der Massageköpfe (1, 2, 4, 5) konstruiert sind, und
die Buchsen in den seitlichen Teilen jades der Rippenbereiche angeordnet sind;
die drei Rippenbereiche, zusammen mit den in den Buchsen befestigten Massageköpfen (1, 2, 4, 5), Massageflächen bilden, die den Positionen der massierten Organe des Rückens in den drei unterschiedlichen Wirbelsäulenbereichen entsprechen, und wobei
die verdickten Enden des Hauptteils (3) buchsen aufweisen, wo Handgriffe und/oder Stecker (6) befestigt werden können.

2. Massagegerät nach Anspruch 1, wobei die Buchssen Massageköpfe (1, 2, 4, 5) mit unterschiedlichem Elastizitätskoeffizienten halten können.

3. Massagegerät nach Anspruch 1, wobei die Buchssen Massageköpfe (1, 2, 4, 5) können, bei denen jede Seite eine unterschiedliche Geometrie aufweist.

4. Massagegerät nach Anspruch 1, das an den von den verdickten Enden des Hauptteils (3) getragenen Buchsen befestigte Stecker (6) aufweist, wobei die Stecker (6) 10aus elastischem Material bestehen.

5. Massagegerät nach Anspruch 1, wobei die Handgriffe jeweils mindestens 20 cm lang und mit den Händen greifbar sind.

6. Massagegerät nach Anspruch 1, wobei das Hauptteil (3) hohl ausgebildet ist, während die Buchsen an den verdickten Enden die Wand durchqueren und mit dem Hohlraum im Hauptteil (3) verbunden sind, damit ein Seil das Hauptteil (3) durchlaufen kann, dessen Enden Laschen für die Hände aufweisen und, von den Buchsen zur Lasche gemessen, mindestens 20 cm lang sind.

7. Massagegerät nach Anspruch 1, wobei das Hauptteil (3) hohl ausgebildet und im Hohlraum ein Permanentmagnet installiert ist.

8. Massagegerät nach Anspruch 1, wobei das Hauptteil (3) hohl ausgebildet und eine Vibrationseinrichtung im Hohlraum angeordnet ist, die das Hauptteil (3) des Massagegeräts vibrieren lassen kann.

9. Massagegerät nach Anspruch 1, wobei das Hauptteil (3) hohl ausgebildet ist und eine Vorrichtung, die wechselseitige Vibrationen der Massageköpfe (1, 2, 4, 5) verursacht, im Hohlraum installiert ist.

10. Massagegerät nach Anspruch 1, das mit Massageköpfen (1, 2, 4, 5) unterschiedlicher Form, Größe und/oder mit unterschiedlichen Elastizitätskoeffizienten versehen ist.

## Revendications

1. Système de massage des muscles du dos d'au moins les trois zones vertébrales suivantes : cervicale, thoracique et lombaire, comprenant :
un corps (3) ayant des extrémités et trois côtés, chacun desdits côtés garantissant l'installation du système de massage sur une surface horizontale, tandis que les plans tangentiels à chacun desdits côtés et qui coïncident avec ladite surface horizontale, qui garantit l'installation de chacun des côtés, forment les uns avec les autres un angle double de 60±15 degrés, la somme totale desdits angles doubles étant de 180 degrés,
lesdits côtés dudit corps (3) étant conjugués les uns avec les autres à l'aide de trois surfaces rainurées, qui portent chacune des parties latérales destinées aux zones paravertébrales du dos et une partie médiane destiné à la colonne vertébrale, qui se trouvent le long de l'axe longitudinal du corps (3) par rapport aux parties latérales ;
**caractérisé en ce que** le système de massage comprend en outre
des têtes de massage détachables (1, 2, 4, 5), dans lequel
le corps (3) est muni de fiches conçues pour fixer lesdites têtes de massage (1, 2, 4, 5), et
lesdites fiches sont situées dans les parties latérales de chacune desdites zones rainurées ;
lesdites trois zones rainurées, avec lesdites têtes de massage (1, 2, 4, 5) fixées dans les fiches, forment des surfaces de massage qui correspondent aux positions des parties massées du dos dans les trois zones différentes de la colonne vertébrale ;
et dans lequel
les extrémités du corps (3) portent des fiches dans lesquelles des poignées et/ou des connecteurs (6) peuvent être insérés.

2. Système de massage selon la revendication 1, dans lequel lesdites fiches peuvent recevoir des têtes de massage (1, 2, 4, 5) de différents coefficients d'élasticité.

3. Système de massage selon la revendication 1, dans lequel lesdites fiches peuvent recevoir des têtes de massage (1, 2, 4, 5) à géométrie différente de chaque côté.

4. Système de massage selon la revendication 1, qui possède des connecteurs (6) insérés au niveau des fiches portées par les extrémités du corps (3), dans lequel les connecteurs (6) sont composés d'un matériau élastique.

5. Système de massage selon la revendication 1, dans lequel lesdites poignées mesurent au moins 20 cm de long chacune et peuvent être attrapées avec les mains.

6. Système de massage selon la revendication 1, dans lequel ledit corps (3) est creux, tandis que lesdites fiches situées sur les extrémités traversent la paroi et se raccordement à la cavité dans le corps (3), de sorte qu'un câble puisse être passé par le corps (3), dont les extrémités sont équipées d'ergots pour les mains et mesurent au moins 20 cm de long, entre les fiches et les ergots.

7. Système de massage selon la revendication 1, dans lequel ledit corps (3) est creux et un aimant permanent est installé dans la cavité.

8. Système de massage selon la revendication 1, dans lequel ledit corps (3) est creux et un dispositif vibrant est placé dans la cavité, afin de faire vibrer le corps (3) du système de massage.

9. Système de massage selon la revendication 1, dans lequel ledit corps (3) est creux, et un dispositif qui provoque des vibrations réciproques desdites têtes de massage (1, 2, 4, 5) est installé dans la cavité.

10. Système de massage selon la revendication 1, équipé d'un groupe de têtes de massage (1, 2, 4, 5) de forme, de taille et/ou de coefficients d'élasticité différents.
